# EUROPEAN PATENT APPLICATION

(11) **EP 3 590 935 A1**
(43) Date of publication of application: **08.01.2020**
(21) Application number: 18182128.1
(22) Date of filing: 06.07.2018
(51) Int. Cl.: C07D 403/06, A61K 31/497, A61P 35/00

(54) **FUSED IMIDAZOLIUM DERIVATIVES FOR USE IN THE TREATMENT OF COLORECTAL CANCER**

(71) Applicant: Deutsches Krebsforschungszentrum, Stiftung des öffentlichen Rechts, 69120 Heidelberg (DE)
(72) Inventor: Zhan, Tianzuo, 68259 Mannheim (DE); Rauscher, Benedikt, 69126 Heidelberg (DE); Faehling, Verena, 69121 Heidelberg (DE); Boutros, Michael, 69117 Heidelberg (DE)
(74) Representative: Kuttenkeuler, David

(57) **Abstract**

The present invention pertains to fused imidazolium derivatives, in particular 11-(2-methoxyethyl)-2-methyl-4,9-dioxo-3-(pyrazin-2-ylmethyl)-4,9-dihydro-1H-naphtho(2,3-d)imidazolium bromide for use in the treatment of colorectal cancer (CRC). The invention provides the therapeutic application of such compounds in a patient subgroup of CRC, which is characterized by a consensus molecular subtype (CMS) 1.

## Description

### FIELD OF THE INVENTION

The present invention pertains to fused imidazolium derivatives, in particular 11-(2-methoxyethyl)-2-methyl-4,9-dioxo-3-(pyrazin-2-ylmethyl)-4,9-dihydro-1H-naphtho(2,3-d)imidazolium bromide, for use in the treatment of colorectal cancer (CRC). The invention demonstrates the therapeutic application of such compounds in a patient subgroup of CRC, which is characterized by a consensus molecular subtype (CMS) 1.

### DESCRIPTION

Colorectal cancer (CRC) is one of the leading causes of cancer related mortality worldwide (Torre et al., 2015). A major challenge in the treatment of CRC is the divergent drug response, which is mainly attributed to tumour heterogeneity (Alizadeh et al., 2015). To segregate CRC into subtypes with distinct responsiveness to antineoplastic drugs, many genetic and protein markers have been screened (Sinicrope et al., 2016). However, only two molecular markers are currently used to guide therapy selection in CRC, including mutations in the Ras oncogenes (Karapetis et al., 2008) and microsatellite instability (Le et al., 2015).

Beyond classifications that rely on genomic alterations, many studies proposed an expression-based classification of CRC. In 2015, a consensus on molecular subtypes was developed by an international consortium that integrated data from different transcriptome-based classifiers (Guinney et al., 2015). The four identified consensus molecular subtypes (CMS) show distinct activation levels of cancer pathways and characteristic genomic alterations. Furthermore, they differ in their clinical properties, such as tumour location or long-term outcome. Hence, integration of CMS has been proposed for future clinical trials (Van Cutsem et al., 2016). Recently, the CMS classification was modified and applied to CRC cell lines, with the aim to identify subtype specific vulnerabilities in preclinical models (Linnekamp et al., 2018; Sveen et al., 2017). In fact, CMS specific effects of HSP90 inhibitors (Linnekamp et al., 2018) and oxaliplatin (Sveen et al., 2017) could be identified, highlighting the potential of transcriptome based response prediction.

EP 1 256 576 A1 discloses fused imidazolium derivatives, applicable for cancer therapy, as well as methods for their synthesis. The disclosure of EP 1 256 576 A1 is incorporated herein by reference in its entirety.

EP 1 614 686 A1 1-(2-methoxyethyl)-2-methyl-4,9-dioxo-3-(pyrazin-2-ylmethyl)-4,9-dihydro-1H-naphtho[2,3-d]imidazol-3-ium bromide which is a novel compound useful as medicines, particularly to its crystals, as well as methods for its synthesis. The disclosure of EP 1 614 686 A1 is incorporated herein by reference in its entirety.

It is an object of the present invention to provide new therapeutic options for the treatment of colorectal cancer. Another object of the present invention is to improve CRC therapy by differential treatment of patient groups suffering from CRC of a specific consensus molecular subtype (CMS) with compounds effective in such CMS.

The problem is solved in a first aspect by a compound for use in the treatment of a colorectal cancer (CRC) in a subject, wherein the compound is a fused imidazolium derivative represented by the following general formula (I), and solvates, salts, stereoisomers, complexes, polymorphs, crystalline forms, racemic mixtures, diastereomers, enantiomers, tautomers, isotopically labelled forms, prodrugs, and combinations thereof, 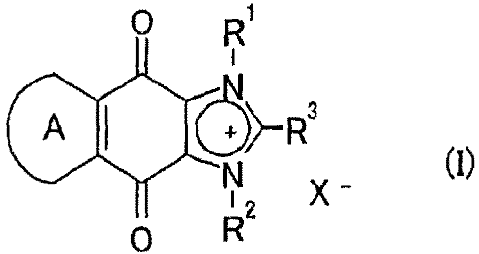 wherein
R¹ and R²: the same or different from each other and each represents -lower alkyl having one or more substituents selected from group B, -lower alkenyl having one or more substituents selected from group B, -lower alkynyl having one or more substituents selected from group B, -RinD, -lower alkyl, -lower alkenyl or -lower alkynyl, with the proviso that at least one of R¹ and R² is -lower alkyl having one or more substituents selected from group B, -lower alkenyl having one or more substituents selected from group B, -lower alkynyl having one or more substituents selected from group B, -cycloalkyl having one or more substituents or -five- to seven-membered saturated heterocyclic ring which may have one or more substituents,
group B: -OR^{a}, -SR^{a}, -prodrug-formed OH, -O-lower alkylene-OR^{a}, -O-lower alkylene-O-lower alkylene-OR^{a}, -O-lower alkylene-NR^{a}R^{b}, -O-lower alkylene-O-lower alkylene-NR^{a}R^{b}, -O-lower alkylene-NR^{c}-lower alkylene-NR^{a}R^{b}, -OCO-NR^{a}R^{b}, -SOR^{a}, -SO₂R^{a}, - SO₂NR^{a}R^{b}, -NR^{a}-SO₂R^{b}, -CO₂H, -NR^{a}R^{b}, -NR^{c}-lower alkylene-NR^{a}R^{b}, -N(-lower alkylene-NR^{a}R^{b})₂, -RinD, -NO₂, -CN, -halogen, -CO₂R^{a}, -COO-, -CONR^{a}R^{b}, -CONR^{a}-O-R^{b}, -NR^{a}-COR^{b}, -NR^{a}-CO-NR^{b}R^{c}, -OCOR^{a} and -CO-R^{a},
R^{a}, R^{b} and R^{c}: the same or different from one another and each represents -H, -lower alkyl, -lower alkylene-RinD or -RinD,
RinD: -five- to seven-membered saturated heterocyclic ring which may have one or more substituents, -cycloalkyl which may have one or more substituents, -cycloalkenyl which may have one or more substituents, -aryl which may have one or more substituents or -heteroaryl which may have one or more substituents,
R³: -H or -lower alkyl which may have one or more substituents, or R² and R³ may together form a lower alkylene having from 2 to 5 carbon atoms which may be interrupted with O, S or NR⁴ (R⁴: -H or -lower alkyl),
ring A: aryl ring which may have one or more substituents or heteroaryl ring which may have one or more substituents, and
X-: counter anion, with the proviso that X- does not exist when the substituent -COO- of the group B forms intramolecular salt with imidazolium cation.
For formulae (I), (II) and (III) above, the following definitions apply.

The term "lower" means a straight or branched form of hydrocarbon chain having up to 6 carbon atoms. The lower (C₁-C₆) alkyl is preferably an alkyl group having from 1 to 4 carbon atoms, and its particularly preferred examples include methyl, ethyl, n-propyl, isopropyl, n-butyl and isobutyl groups. The preferred examples of lower (C₂-C₆) alkenyl include vinyl, allyl, 1-propenyl, isopropenyl, 1-butenyl, 2-butenyl and 3-butenyl groups. The preferred examples of lower (C₂-C₆) alkynyl include ethynyl, 1-propynyl, 2-propynyl, 1-butynyl, 2-butynyl, 3-butynyl and 1-methyl-2-propynyl groups. The preferred examples of lower alkylene include methylene, ethylene, trimethylene and 2,2-dimethyltrimethylene groups.

The term "aryl" means an aromatic hydrocarbon ring group having from 6 to 14 carbon atoms, more preferably phenyl, naphthyl and fluorenyl groups.

The term "heteroaryl" means five- or six-membered monocyclic heteroaryl groups containing from 1 to 4 hetero atoms selected from N, S and O and bicyclic heteroaryl groups in which they are fused with a benzene ring or a said monocyclic heteroaryl ring, which may be partially saturated. Also, when it contains N atom, it may form N-oxide. Furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidinyl, pyridazinyl, pyrazinyl and triazinyl groups are preferred as the five- or six-membered monocyclic heteroaryl, and benzofuranyl, benzothienyl, benzothiadiazolyl, benzothiazolyl, benzoxazolyl, benzoxadiazolyl, benzoimidazolyl, indolyl, isoindolyl, indazolyl, quinolyl, isoquinolyl, cinnolinyl, quinazolinyl, quinoxalinyl, benzodioxolyl, indolizinyl and imidazopyridyl groups are preferred as the bicyclic heteroaryl. As the partially saturated heteroaryl, 1,2,3,4-tetrahydroquinolyl group and the like can be exemplified. Further preferred are furyl, thienyl, imidazolyl, pyridyl, pyrazinyl, pyrimidinyl, pyridadinyl, indolyl, benzoimidazolyl, benzodioxonyl and quinolyl groups, and particularly preferred are pyridyl, pyrazinyl and pyrimidinyl groups.

The term "cycloalkyl" means cycloalkyl groups having from 3 to 10 carbon atoms and particularly preferred are cyclopropyl, cyclopentyl, cyclohexyl and adamantyl groups. The cycloalkenyl groups have from 3 to 8 carbon atoms and particularly preferred are cyclopentenyl and cyclohexenyl groups.

As the "counter anion", there is no particular limitation with the proviso that it is a pharmaceutically acceptable anion as a counter anion of imidazolium cation, and its preferred examples include monovalent or divalent anions such as halogen ions, organic sulfonate ions (methanesulfonate ion, ethanesulfonate ion, benzenesulfonate ion, toluenesulfonate ion and the like), acetate ion, trifluoroacetate ion, carbonate ion, sulfate ion and the like, of which halogen ions are particularly preferred.

As the "halogen", F, Cl, Br and I atoms can be exemplified, and the "halogen ion" means their ions. The "halogeno lower alkyl" is the aforementioned lower alkyl which is substituted by one or more of the halogens, and is preferably -CF₃.

The term "saturated heterocyclic" means a five- to seven-membered monocyclic saturated heterocyclic ring containing from 1 to 4 hetero atoms selected from N, S and O, or its crosslinked ring. Preferred are tetrahydropyranyl, tetrahydrofuranyl, pyrrolidinyl, piperazinyl, azepanyl, diazepanyl, quinuclidinyl, piperidyl and morpholinyl groups.

Where a group has one or more substituents selected from group C, the number of said substituents is not particularly limited, but is preferably from 1 to 4.

Among group C, more preferred are -lower alkyl, -halogen, -halogeno lower alkyl, -OH, -O-lower alkyl, -O-lower alkylene-OH, -O-lower alkylene-O-lower alkyl, -lower alkylene-NH₂, - NH2, -NH-lower alkyl, -N(lower alkyl)₂, -CO₂H, -CO₂-lower alkyl, -CO-NH₂, -SO₂-NH₂, - NO₂and -CN. For ring A, a particularly preferred substituent is -NO₂.

Where R³ is lower alkyl which may have one or more substituents from group B, -halogen, - OR^{a}, -SR^{a}, -NR^{a}R^{b}, -NO₂ or -CN are preferred substituents.

As the groups using R^{a}, R^{b} and R^{c} in the aforementioned groups B and C, those in which R^{a}, R^{b} and R^{c} are -H or -lower alkyl are more desirable.

In general formula (I), the term "R² and R³ together form alkylene having from 2 to 5 carbon atoms which may be interrupted with O, S or NR⁴ where R⁴ is -H or -lower alkyl" means that R² and R³ together form a C2 to C₅ alkylene chain which may be interrupted with O, S or NR⁴(preferably -(CH₂)₄-, -(CH₂)₂OCH₂- or -(CH₂)₂N(Me)CH₂-), and combine with the adjacent N and C atoms to form a (e.g. four- to seven-membered)hetero ring which is fused with the imidazole ring.

Preferred compounds (I) of the invention are those where at least one of R¹ and R² is lower alkyl having one or more substituents selected from -OR^{a}, -O-lower alkylene-OR^{a}, -O-lower alkylene-O-lower alkylene-OR^{a}, -(saturated heterocyclic which may have one or more substituents selected from group C), -(aryl which may have one or more substituents selected from group C) and -(heteroaryl which may have one or more substituents selected from group C); or at least one of R¹ and R² is lower alkyl substituted by a heteroaryl group selected from furyl, thienyl, imidazolyl, pyridyl, pyrazinyl, pyrimidinyl, pyridazinyl, indolyl, benzoimidazolyl, benzodioxonyl and quinolyl group, which may have one or more substituents selected from group C; or
one of R¹ and R² is lower alkyl substituted by -O-lower alkyl and the other is lower alkyl substituted by one substituent selected from
-O-lower alkylene-O-lower alkyl, -O-lower alkylene-O-lower alkylene-O-lower alkyl, - (aryl which may have one or more substituents selected from group C), -(heteroaryl which may have one or more substituents selected from group C) and -O-lower alkyl; or
at least one of R¹ and R² is lower alkyl having one substituent selected from - (heteroaryl selected from pyridyl, pyrazinyl and pyrimidinyl, which may have one or more substituents selected from the group C), -O-lower alkylene-O-lower alkyl and -O-lower alkyl; or
R³ is a methyl group; or
ring A is a benzene ring which may be substituted by -NO₂; or,
for formula (I), X⁻ is a halogen ion.

Also preferred are compounds wherein at least one of R¹ and R² is lower alkyl having one substituent selected from -(heteroaryl selected from pyridyl, pyrazinyl and pyrimidinyl, which may have one or more substituents selected from group C), -O-lower alkylene-O-lower alkyl and -O-lower alkyl, and ring A is a benzene ring which may be substituted by - NO₂.

Among compounds (I) of the invention, particularly preferred compounds are 1-[(6-chloro-3-pyridyl)methyl]-3-(2-methoxyethyl)-2-methyl-4,9-dioxo-4,9-dihydro-1H-naphtho[2,3-d]imidazol-3-ium, 1,2-dimethyl-4,9-dioxo-3-[(2-tetrahydrofuranyl)methyl]-4,9-dihydro-1H-naphtho[2,3-d]imidazol-3-ium, 1,3-bis(2-methoxyethyl)-2-methyl-4,9-dioxo-4,9-dihydro-1H-naphtho[2,3-d]imidazol-3-ium, 3-(2-methoxyethyl)-2-methyl-4,9-dioxo-1-(2-pyrazinylmethyl)-4,9-dihydro-1H-naphtho[2,3-d]imidazol-3-ium, 1-[3-(1H-4-imidazolyl)propyl]-3-(2-methoxyethyl)-2-methyl-4,9-dioxo-4,9-dihydro-1H-naphtho[2,3-d]imidazol-3-ium, 3-(2-methoxyethyl)-2-methyl-1-[(5-methyl-2-pyrazinyl)methyl]-4,9-dioxo-4,9-dihydro-1H-naphtho[2,3-d]imidazol-3-ium, 2-methyl-4,9-dioxo-1,3-bis(2-pyrazinylmethyl)-4,9-dihydro-1H-naphtho[2,3-d]imidazol-3-ium, 1-[2-(2-methoxyethoxy)ethyl]-3-(2-methoxyethyl)-2-methyl-4,9-dioxo-4,9-dihydro-1H-naphtho[2,3-d]imidazol-3-ium, 1-{2-[2-(2-methoxyethoxy)ethoxy]ethyl}-3-(2-methoxyethyl)-2-methyl-4,9-dioxo-4,9-dihydro-1H-naphtho[2,3-d]imidazol-3-ium, 1-(2-methoxyethyl)-2-methyl-4,9-dioxo-3-(3-pyridylmethyl)-4,9-dihydro-1H-naphtho[2,3-d]imidazol-3-ium, 3-(2-methoxyethyl)-2-methyl-4,9-dioxo-1-(2-pyridylmethyl)-4,9-dihydro-1H-naphtho[2,3-d]imidazol-3-ium, 3-(2-methoxyethyl)-2-methyl-4,9-dioxo-1-(4-pyridylmethyl)-4,9-dihydro-1H-naphtho[2,3-d]imidazol-3-ium, 1-[(2-chloro-3-pyridyl)methyl]-3-(2-methoxyethyl)-2-methyl-4,9-dioxo-4,9-dihydro-1H-naphtho[2,3-d]imidazol-3-ium, 1-[(2-hydroxy-4-pyridyl)methyl]-3-(2-methoxyethyl)-2-methyl-4,9-dioxo-4,9-dihydro-1H-naphtho[2,3-d]imidazol-3-ium, 3-(2-methoxyethyl)-1-[(6-methoxy-3-pyridyl)methyl]-2-methyl-4,9-dioxo-4,9-dihydro-1H-naphtho[2,3-d]imidazol-3-ium, 1-[(2-chloro-4-pyridyl)methyl]-3-(2-methoxyethyl)-2-methyl-4,9-dioxo-4,9-dihydro-1H-naphtho[2,3-d]imidazol-3-ium, 1-(4-chlorobenzyl)-3-(2-methoxyethyl)-2-methyl-4,9-dioxo-4,9-dihydro-1H-naphtho[2,3-d]imidazol-3-ium, 1-(4-fluorobenzyl)-3-(2-methoxyethyl)-2-methyl-4,9-dioxo-4,9-dihydro-1H-naphtho[2,3-d]imidazol-3-ium and 1,3-bis(2-methoxyethyl)-2-methyl-5-nitro-4,9-dioxo-4,9-dihydro-1H-naphtho[2,3-d]imidazol-3-ium, or tautomers thereof and their salts with halogen ions.

The compound (I) of the invention exists in tautomer forms represented by the following formula due to delocalization of the cation, and these isomers in separated forms or mixtures thereof are included in the invention. The compound mentioned herein as 1H-imidazol-3-ium derivative includes 3H-imidazol-1-ium derivative as its tautomer and mixture of both isomers. In this connection, X⁻ does not exist when the compound (I) has a substituent -COO⁻ and forms intramolecular salt with the imidazolium cation.

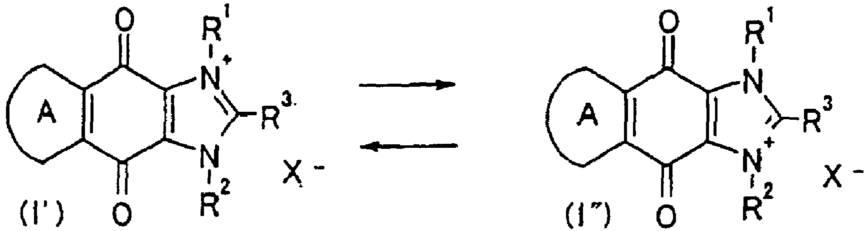

In addition to the aforementioned salt with a counter anion, the compound (I) of the invention forms other salts in some cases depending on the kinds of substituents, and these salts are also included in the invention. Though these salts are not particularly limited with the proviso that they are pharmaceutically acceptable salts, acid addition salts with an inorganic acid (hydrochloric acid, hydrobromic acid, hydroiodic acid, sulfuric acid, nitric acid, phosphoric acid and the like) and with an organic acid (formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid, glutamic acid and the like) can be cited as illustrative examples of acid addition salts, and salts with an inorganic base containing a metal (sodium, potassium, magnesium, calcium, aluminum and the like) or with an organic base (methylamine, ethylamine, ethanolamine, lysine, ornithine and the like) and ammonium salts and the like can be exemplified as salts with bases.

Depending on the kinds of substituents, geometrical isomers and tautomers exist in the compound (I) of the invention in some cases, and these isomers in separated forms or mixture thereof are included in the invention. Also, since certain compounds of the invention have asymmetric carbon atom, isomers based on the asymmetric carbon atom can exist. The invention includes mixed and separated forms of these optical isomers. Also, compounds of the invention may sometimes form N-oxide depending on the kinds of substituents, and these N-oxide compounds are also included in the invention. In addition, various hydrates and solvates and polymorphic substances of the compound (I) of the invention are also included in the invention.

A preferred fused imidazolium derivative for use according to the invention is the above compound, wherein A is an unsubstituted aryl.

Another preferred fused imidazolium derivative for use according to the invention is the above compound, wherein R¹ is -CH2(Pyrazin).

Another preferred fused imidazolium derivative for use according to the invention is the above compound, wherein R² is -(Ch₂)₂OMe.

Another preferred fused imidazolium derivative for use according to the invention is the above compound, wherein R³ is methyl.

Another preferred fused imidazolium derivative for use according to the invention is the above compound, wherein X is halogen, preferably Br.

Most preferably the fused imidazolium derivative for use according to the invention is 11-(2-methoxyethyl)-2-methyl-4,9-dioxo-3-(pyrazin-2-ylmethyl)-4,9-dihydro-1H-naphtho(2,3-d)imidazolium bromide (YM-155 or SEPANTRONIUM BROMIDE), preferably of the formula (II).

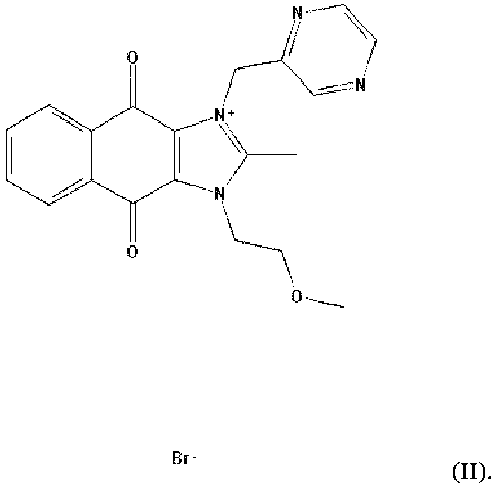

Methods for synthesizing the fused imidazolium derivatives of the invention are disclosed in EP 1 256 576 A1 between and including paragraphs 39 to 60, which shall be incorporated herein by reference.

The methods for producing the compound according to formula (II), and derivatives thereof, is in detail disclosed in EP 1 614 686 A1 between and including paragraphs 16 to 20, which shall be incorporated herein by reference. Other therapeutically active salts of the compound shall be included in the present disclosure.

Without wishing to be bound by the theory, it is believed that YM155 functions to knockdown survivin by binding to the transcription factor interleukin enhancer-binding factor 3 (ILF3). This transcription factor normally binds to p54mb to form a complex, which binds the survivin promoter to promote transcription of the survivin gene. However, it is believed that YM155 binds the ILF3-p54mb complex and prevents that complex from binding the survivin promoter. (Yamauchi et al.). Sepantronium bromide (YM155) induces disruption of the ILF3/p54mb complex, which is required for survivin expression(Biochem Biophys Res Comm 2012; 425: 711-716). YM155 has been evaluated for safety and efficacy in terms of other cancers, such as prostate cancer and although safe, efficacy results are mixed (See, e.g., Lewis et al., A multi-center phase II evaluation of the small molecule surviving suppressor YM155 in patients with unresectable stage III or IV melanoma, Invest. New Drugs (2011 29:161-166 and Tolcher et al, Phase I and pharmacokinetic study of YM155, a small-molecule inhibitor of survivin (2008) J. Clin. Oncology 26(32):5198-5203).

The term "colorectal cancer" or "CRC" refers to a malignant neoplasm of the large intestine/colon within a given subject, wherein the neoplasm is of epithelial origin and is also referred to as a carcinoma of the large intestine/colon. According to the invention, colorectal cancer is defined according to its type, stage and/or grade, genetic characteristics and particular according its CMS, which may be determined by transcriptome analysis. Typical staging systems of CRC known to those skilled in the art such as the tumour TNM staging system (the system adopted by the American Joint Committee on Cancer and the International Union Against Cancer). The term "colorectal cancer", when used without qualification, includes both localized and metastasised colorectal cancer. The term "colorectal cancer" can be qualified by the terms "localized" or "metastasised" to differentiate between different types of tumour as those words are defined herein. The terms "colorectal cancer" and "malignant disease of the large intestine/colon" are used interchangeably herein. The term "colorectal cancer" includes, but is not limited to, colon cancer, rectal cancer, and bowel cancer.

In preferred embodiments, the CRC treated according to the invention is a CRC of a particular subtype, more preferably wherein the CRC is a tumour having a deficiency in, or decreased expression of, at least one mismatch repair gene, most preferably wherein the CRC is a tumour of Consensus Molecular Subtype 1 (CMS1).

The determination of CMS may be realized by diagnosing in a sample of the tumour alterations in the expression of components of mismatch repair as assessed by routine pathology, such as a decreased immunohistochemical expression of any one of or a combination of MLH1, MSH2, MSH6, PMS2. Alternatively, or additionally testing MSI by polymerase chain reaction. Another preferred option includes the diagnostic of hypermutation in a sample of the colorectal cancer of the subject to be treated. Such a diagnostic of hypermutation may be determined if in a cell of the CRC of the subject >12 mutations per 106 bp is found, for example as determined by exome capture sequencing.

Most preferred in context of the invention is a determination of CMS by transcriptome analysis of a sample of the tumour of the subject to be treated. Testing of expression from fresh frozen tissue of patients by isolation of total RNA and expression analysis by microarray (platforms Affymetrix or Agilent) or RNA-seq. Classification of tumours according to the R-package "CMSclassifier".

As used herein, the term "transcriptome" can be defined as the collection of transcribed elements of the genome. In addition to mRNAs, it also represents non-coding RNAs which are used for structural and regulatory purposes as well as splice variants, primary transcripts, and intermediates (hnRNA for example). Alterations in the structure or levels of expression of any one of these RNAs or their proteins can contribute to disease. A transcriptome analysis therefore includes the determination of the expression of at least a set of multiple RNA molecules in a sample, preferably a nearly complete (more than 50%, 60%, 70% or most preferably more than 80%, 90%, or more than 95%) or a complete set of all expressed RNA.

The transcriptome can be profiled in accordance with the invention by high throughput techniques including SAGE, microarray, and sequencing of clones from cDNA libraries. For more than a decade, oligo-nucleotide microarrays have been the method of choice providing high throughput and affordable costs and shall be included as a method of choice in accordance with the invention. Another approach for transcriptome analyses is the so called "next generation sequencing" (NGS). Current NGS platforms include the Roche 454 Genome Sequencer, Illumina's Genome Analyzer, and Applied Biosystems' SOLiD. These platforms can analyze tens to hundreds of millions of DNA fragments simultaneously, generate giga-bases of sequence information from a single run, and have revolutionized SAGE and cDNA sequencing technology. For example, the 3' tag Digital Gene Expression (DGE) uses oligo-dT priming for first strand cDNA synthesis, generates libraries that are enriched in the 3' untranslated regions of polyadenylated mRNAs, and produces 20-21 base cDNA tags.

Classification of the tumour as CMS1 to CSM4 is then performed according to Guinney, J. et al. 2015, and preferably using the CSMclassifier package in R, in accordance with Eide et al. 2017. Both publications are included in this disclosure by reference in their entireties.

A treatment in accordance with the herein disclosed invention is preferable in a CRC subgroup which is not CMS2, and most preferably is not any of CMS2, CMS3, and CMS4.

In some preferred embodiments of the invention a treatment of the invention comprises as a first step the stratification of the subject into a patient group suffering from a CRC CMS1, and preferably not CMS2, most preferably not CMS2 to 4. Such method steps for a stratification of a subject suffering from CRC are described herein.

In context of the herein disclosed invention a treatment of a disease comprises the administration of the compound of the invention to a subject in need of such a treatment. Preferably the compound according to the invention is administered to the subject in an amount effective to treat the disease - such as a tumour - in the subject. Such amounts are generally referred to as "therapeutically effective" amounts, and should be understood to refer to an amount of a composition, or agent or compound in the composition, in accordance with the invention, capable of minimising the progression of, treating, preventing recurrence of or ameliorating cancer or the spread (metastasis) thereof. A therapeutically effective amount may be determined empirically and in a routine manner in relation to treating cancer, and will result in increased life expectancy of the subject suffering from the cancer.

An effective dose or dose range is expected to vary for the compounds described herein for any number of reasons, including the molecular weight of the compound, bioavailability in the dosage form, route of administration, solubility of the compound in a given excipient (e.g., carrier or vehicle), specific activity (e.g., EC₅₀), etc. In any case, the effective range (e.g., the therapeutic window) between the minimally-effective dose, and maximum tolerable dose in a patient can be determined empirically by a person of skill in the art, with end points being determinable by *in vitro* and *in vivo* assays, such as those acceptable in the pharmaceutical and medical arts for obtaining such information regarding agents, such as parenteral and/or intravenous compositions. Different concentrations of the agents described herein are expected to achieve similar results. The compounds can be administered one or more times daily, for example two to four times daily, once every two, three, four, five or more days, weekly, monthly, etc., including increments there-between. It is possible to deliver the drug continuously, for instance in severe cases, or substantially continuously. A composition can be administered for duration effective to treat a cancer, preferably CRC, in a patient, such as until a patient is deemed cancer- free, or for a length of time suitable to treat an ordinary patient. A person of ordinary skill in the pharmaceutical and medical arts will appreciate that it will be a matter of design choice and/or optimization to identify a suitable dosage regimen for improving symptoms of a condition.

In non-limiting embodiments, the compounds of the invention, such as YM155, is administered parenterally to a CRC patient in an effective dosage range between about 0.1 mg/kg and about 5 g/kg per day, or an equivalent thereof, for a time period ranging from one day to one year, including any increment there-between, or alternately between about 0.1 mg/m²/d and about 5 mg/m²/d. In embodiments in which the composition is administered continuously, for example intravenously, an exemplary effective range for YM155, or any other compound of the invention, is between about 0.5 or 1 mg/m²/d and about 10 mg/m²/d, 1.8-6.0 mg/m²/d, including 4.8 mg/m²/d and 1, 2, 3, 4, 5, 6, 7, 8, 9 and 10 mg/m²/d (see also Tolcher AW et al. J Clin Oncol. 2008 Nov 10; 26(32): 5198-5203).

Any of the compounds described herein may be compounded or otherwise manufactured into a suitable composition for use, such as a pharmaceutical dosage form or drug product in which the compound is an active ingredient. Compositions may comprise a pharmaceutically acceptable carrier, vehicle or excipient. An excipient is an inactive substance used as a carrier or vehicle for the active ingredients of a medication. Although "inactive," excipients may facilitate and aid in increasing the delivery, stability or bioavailability of an active ingredient in a drug product. Non-limiting examples of useful excipients for parenteral dosage forms include: rheology modifiers, emulsifiers, oils, adjuvants, buffers, salts, acids, bases, diluents, solvents, preservatives, antioxidants, sorbents, vitamins, etc., as are available in the pharmaceutical/compounding arts. In non-limiting embodiments, the dosage form is any dosage form suitable for parenteral or intravenous administration and the composition comprises water, saline (e.g., normal saline, 0.9% w/v NaCl in water), phosphate-buffered saline and other buffered solutions, dextrose, emulsifiers etc., as are broadly known in the pharmaceutical arts. Therapeutic/pharmaceutical compositions are prepared in accordance with acceptable pharmaceutical procedures and practices, such as are described in Remington: The Science and Practice of Pharmacy, 21st edition, Eds. Paul Beringer et al., Lippincott, Williams & Wilkins, Baltimore, MD Easton, Pa. (2005), describing a variety of formulations useful in the methods described herein, such as parenteral and intravenous dosage forms.

In another embodiment any compound as described herein is provided and delivered in a transdermal device. Suitable structures and compositions for such a device are well-known in the pharmaceutical arts, generally including an occluding backing comprising an adhesive on one side to face a patient, and a drug reservoir on the same side of the occluding backing as the adhesive. The reservoir may be a non-woven fabric or gauze, or a hydrogel in which an active agent, such as any composition described herein may be absorbed or admixed. Often a permeation enhancing compound is includes in the reservoir to facilitate transfer of the active agent into/through the skin. See,e.g., Remin ton: The Science and Practice of Pharmacy, 21st edition, Eds. Paul Beringer et al., Lippincott, Williams & Wilkins, Baltimore, MD Easton, Pa. (2005). One non-limiting example of a transdermal device is a microneedle array, such as is disclosed in United States Patent Publication No. 20110098651, incorporated herein by reference.

Additional compounds or compositions may be included in a drug product comprising one or more of the compounds for treating colon cancer. In one embodiment, an additional active agent (drug), that is a compound having a physiological activity, such as a second anti-cancer chemotherapeutic drug is co-administered with or co-formulated with the compound(s) of the invention (that is, in the same dosage form, such as an intravenous solution comprising more than one compound or composition). A detailed description of anti-cancer drugs to be used in context of the present invention is provided herein elsewhere. Non-limiting examples of other compounds or compositions that might be combined with the compositions described herein for use in treating colon cancer: antibiotics, an (second) interfering nucleic acid, analgesics, anti-inflammatories, protein-based therapeutics, such as cytokines or interleukins, antibodies of fragments thereof, an antiemetic, a cell-based therapeutic (e.g., a cell-based immunotherapeutic, such as, without limitation, an engineered dendritic- or T-cell), and/or a vector comprising a gene for producing a therapeutic (e.g, an immunotherapeutic, or antisense therapeutic), such as a plasmid, a recombinant viral nucleic acid or a recombinant viral transducing particle (a viral particle, such as an Adenovirus, Adeno-Associated Virus, retrovirus, etc. transducing particle, carrying a recombinant viral genome in which a desired gene has been placed, for example a transducing virus particle for delivery of an immunotherapeutic, an interfering nucleic acid or a protein-based therapeutic).

Treatment in accordance with the invention are preferably treatments of CRC and constitutes a reduction or halt of CRC tumour growth, or constitutes a reduction of CRC tumour size, or constitutes a complete elimination of CRC tumour in the subject. A complete elimination in context of the invention shall be understood as a reduction of tumour mass or tumour cells in the subject to a level below detectability in the subject.

In further embodiments of the herein disclosed invention a treatment comprises the additional administration of at least one further anti-tumour therapeutic effective for treating colon cancer, preferably CRC. Alternatively or additionally a treatment in accordance with the invention may comprise any other anti-cancer therapeutic option, such as surgery, tumour destruction by physical means such as heat or radiation.

Drugs approved for the treatment of colorectal cancer, and that are preferably used in combination with the compounds and treatments of the invention are selected from Avastin (Bevacizumab), Bavencio (Avelumab), Camptosar (Irinotecan Hydrochloride), , Cyramza (Ramucirumab), Eloxatin (Oxaliplatin), Erbitux (Cetuximab), 5-FU (Fluorouracil Injection), Fusilev (Leucovorin Calcium) Keytruda (Pembrolizumab) Lonsurf (Trifluridine and Tipiracil Hydrochloride), Opdivo (Nivolumab), Stivarga (Regorafenib), Tecentriq (Atezolizumab), Vectibix (Panitumumab), Xeloda (Capecitabine) Yervoy (Ipilimumab) and Zaltrap (Ziv-Aflibercept). Also any combinations of the aforementioned anti-cancer drugs may be used in a treatment in accordance with the invention. Such combinations include, but are not limited to: Capecitabine and Oxaliplatin; Irinotecan Hydrochloride, Fluorouracil and Leucovorin Calcium (Folinic Acid); Irinotecan Hydrochloride, Fluorouracil, Leucovorin Calcium (Folinic Acid) and Bevacizumab or Cetuximab; Leucovorin Calcium (Folinic Acid), Fluorouracil, and Oxaliplatin; Fluorouracil and Leucovorin Calcium; Capecitabine (Xeloda) and Irinotecan Hydrochloride; Capecitabine (Xeloda) and Oxaliplatin.

Another aspect of the present invention pertains to a method for stratifying a subject suffering from CRC, the method comprising the steps of
(a) Providing a biological sample (preferably a sample comprising cells) of the CRC tumour of the subject,
(b) Determining in said sample any of the following:
   (i) The presence or absence of a biomarker in the tumour cells which is selective and/or specific for a CRC tumour of CMS1, and/or
   (ii) The expression, function and/or stability of one or more biomarkers selected from the group consisting of SLC35F2, CCDC22, WASHC4 and SNX17 in the tumour cells,
wherein the absence of a biomarker of (i) and/or a reduced expression, function and/or stability of one or more biomarkers of (ii) indicates an increased resistance of the CRC tumour to a treatment with a compound of the present invention. A treatment in this context shall be understood to be a treatment of the invention as described herein elsewhere.

In the context of the present invention, the term "stratifying" or "stratification" relates to the identification of a group of patients with shared "biological" or "pathological" characteristics by using molecular and biochemical diagnostic testing to select the optimal treatments for the patients. A stratification of CRC patients in context of the herein disclosed invention preferably relates to the identification of a CMS subgroup of a CRC in a patient.

A biological sample in context of the invention is preferably a sample containing cells of a tumour or cells derived from the tumour vicinity. Biological samples of the invention can be tumour biopsies, or a tissue sample from a resected tumour. Further possibilities include any other tissue or liquid sample derived from a patient such as a urine or stool sample, a blood sample, including serum and plasma samples. Any sample obtained from a patient which can be suspected to include informative material such as tumour cells is suitable in context of the herein disclosed invention.

Preferably the method of the invention includes the determination of gene expression of one or more genes selective for a CRC subtype selected from CMS1 to 4 in order to obtain information about the CMS identity of the tumour of the patient. Gene expression may be determined in various ways known in the art, including but not limited to, immunological based methods, PCR based techniques, deep sequencing approaches, microarray approaches, or any other methodology known to the skilled person that provides information about the expression of a given gene or genetic element.

Yet, another aspect of the invention pertains to a method of identifying whether a patient subgroup suffering from a disorder benefits from a treatment with a candidate compound, the method comprising the steps of:
(a) Providing *in vitro* data on the effectiveness of a candidate compound as a therapeutic against a plurality of different cultured cell lines involved with the disorder,
(b) Assigning one or more predetermined consensus molecular subtype(s) of the disorder to each of the different cell lines in (a),
wherein a significant association of the effectiveness of a candidate compound as a therapeutic with a specific predetermined consensus molecular subtype in the plurality of cultured cell lines indicates that a patient subgroup distinguished by said associated predetermined consensus molecular subtype will benefit from a treatment with said candidate compound.

Candidate compounds in context of this invention may include, for example, small molecules such as small organic compounds (e.g. , organic molecules having a molecular weight between about 50 and about 2,500 Da), peptides or mimetics thereof, ligands including peptide and non- peptide ligands, polypeptides, proteins, such as antibodies or T cell receptors, as well as derivatives thereof, protein-complexes, nucleic acid molecules such as aptamers, interfering RNA, such as siRNA, shRNA, gene editing components including guide RNA and DNA, sgRNA, etc. optionally in combination with gene editing nucleases and expression constructs thereof, peptide nucleic acid molecules, and components, combinations, and derivatives thereof. Many more categories of molecular compounds having an impact in biological systems are known to the skilled artisan and may be used as candidate compounds in the screening approaches of the present invention.

A disorder for which a therapeutic is screened in accordance with the screening method of the invention is preferably a proliferative disorder, such as cancer, and preferably is CRC.

In some embodiments the *in vitro* data is data on the effectiveness of the candidate compound to impair cell viability and/or growth and/or invasiveness of said *in vitro* cell lines.

Preferably, the consensus molecular subtype is characterized by a differential expression of one or more genes in the cells involved with the disorder compared to at least one other such consensus molecular subtypes of the disorder. A preferred example of such consensus molecular subtypes are the herein mentioned CMS1 to CMS4 of CRC.

In this context, in a further aspect of the invention, there is provided the above described treatment which in addition comprises a prior step of stratifying the subject to be treated by determining the CMS of the CRC tumour in the subject. For example according to the above described stratification method. Preferably, stratifying involves obtaining a cellular sample of the CRC tumour of the subject and determining in said sample an expression level of one or more biomarkers selective and/or specific for a CRC tumour of CMS1.

In other embodiments, stratifying involves obtaining a cellular sample of the CRC tumour and determining in said sample the expression, function and/or stability of one or more biomarkers selected from the group consisting of SLC35F2, CCDC22, WASHC4 and SNX17.

The present invention will now be further described in the following examples with reference to the accompanying figures and sequences, nevertheless, without being limited thereto. For the purposes of the present invention, all references as cited herein are incorporated by reference in their entireties. In the Figures:

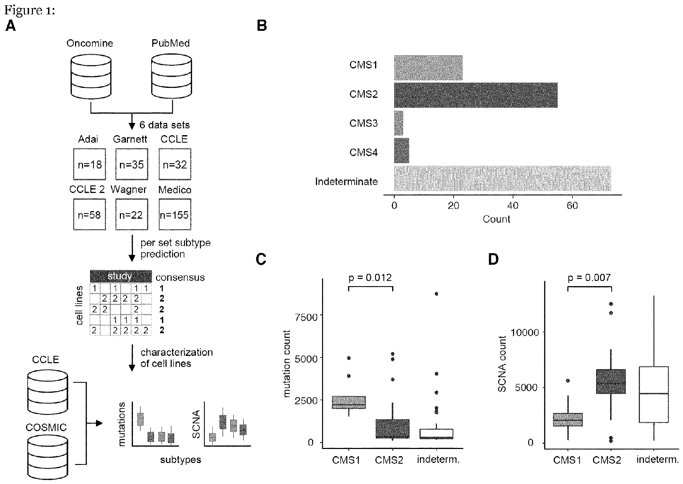

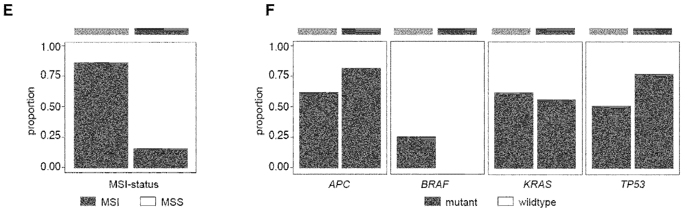

**Figure 1****: Classification of colorectal cancer cell lines into consensus molecular subtypes (CMS).**
   (A) Workflow of data collection and subtype classification. Expression data of CRC cell lines were retrieved from six publicly available datasets. For each dataset, the CMS of every cancer cell line was separately identified using the R package 'CMSclassifier'. The CMS that was determined by the majority of datasets was then considered as the consensus. Finally, genomic data from CCLE and COSMIC were used to analyse CMS specific distributions of mutations and copy number alterations.
   (B) Distribution of CRC cell lines to different CMS. The majority of classified cell lines are assigned to CMS1 or CMS2.
   (C-F) Cancer cell lines and primary tumours of the same CMS share similar genomic features. Mutation count is higher in CMS1 cell lines (C) whereas somatic copy number alteration (SCNA) count is higher CMS2 cell lines (D). Distribution of microsatellite instability (E) and mutations in key cancer genes (F) is similar to primary CRC tissue.

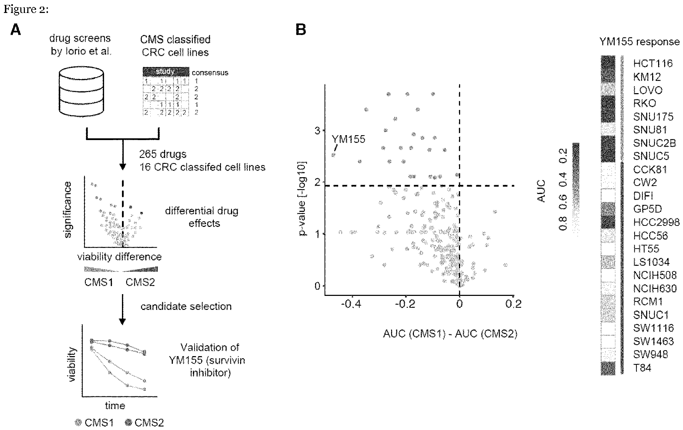

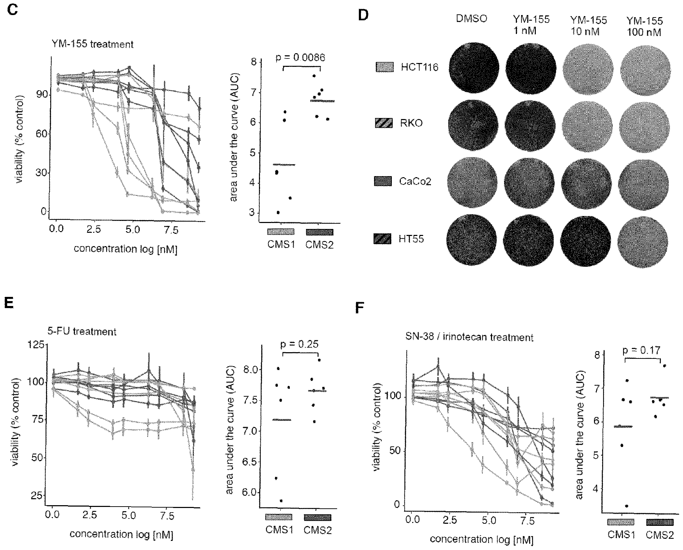

**Figure 2****: CMS1 colorectal cancer shows increased vulnerability to the survivin inhibitor YM155.**
   (A) Overview showing combination of CMS classification and drug response data from screens to identify novel subtype specific vulnerabilities. Drug response data from a large screen performed by Iorio et al. was combined with the CMS classification of CRC cell lines to identify differential drug effects between CMS1 and CMS2 cell lines. The selected candidate was then independently validated.
   (B) Analysis of drug response data for 8 CMS1 and 16 CMS2 CRC cell lines identifies YM155 as a compound with selective effect on CMS1 cancer (left). Heatmap showing the viability effect of YM155 on individual CMS1 and CMS2 cell lines within the screen (blue indicates a low viability as measured by the AUC).
   (C-D) Validation of YM155 as a selective drug in CMS1 colorectal cancer. Cell lines were treated with increasing drug concentrations for 72 h. Dose response curves were determined for 6 CMS1 and 6 CMS2 cell lines and comparison of AUCs between groups show a significantly higher vulnerability of CMS1 colorectal cancer (C). Long-term proliferation assays reveal an approximately 100-fold higher sensitivity of CMS1 cell lines HCT116 and RKO. A representative result of three independent replicates is shown.
   (E-F) 5-fluorouracil and SN-36/irinotecan do not show a different viability effect in CMS1 and CMS2 colorectal cancer. Dose response curves were determined for CMS1 and CMS2 colorectal cancer and comparison of AUCs does not demonstrate a significant difference between CMS1 and CMS2. (C, E-F) Data are shown as mean value ± s.e.m. of 3-4 independent replicates.

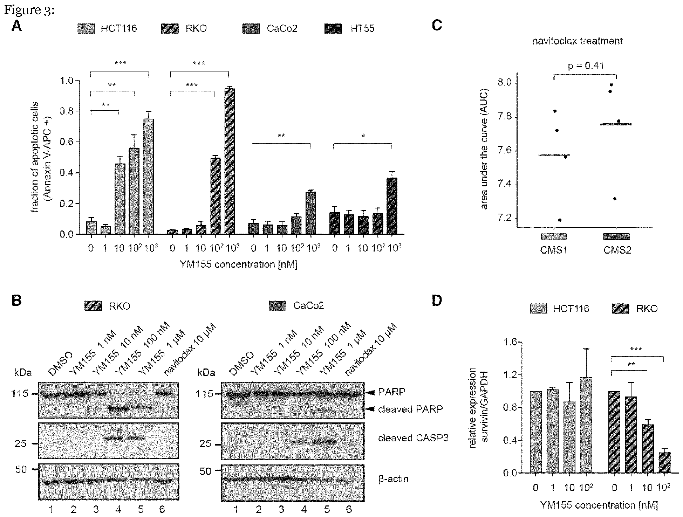

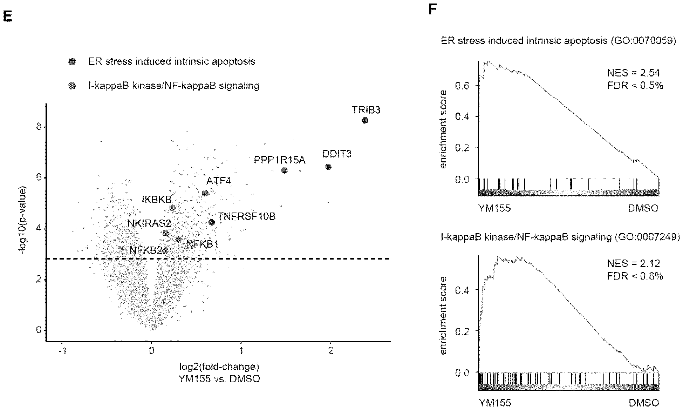

**Figure 3****: YM155 preferentially induces apoptosis in CMS1 cells**
   (A-B) CMS1 cell lines are more sensitive towards YM155 induced apoptosis. The CMS1 cell lines RKO and HCT116 and the CMS2 cell lines HT55 and CaCo2 were treated with increasing concentrations of YM155. Induction of apoptosis was measured by FACS after staining with APC Annexin V and 7-AAD. Apoptosis can be measured at lower concentrations in CMS1 cell lines (A). Data are shown as mean value ± s.e.m. of 3-4 independent replicates. Cell lysates were additionally analyzed by PARP and CASP8 immunoblot. β-actin serves as a loading control. Cleavage of PARP and CASP8 was detected at low concentrations in CMS1 but not CMS2 cell lines (B). A representative blot of three independent replicates is shown.
   (C) CMS1 CRC lines do not show increased vulnerability to the apoptosis inducing drug navitoclax. CMS1 and 2 cell lines were treated with increasing concentrations of the Bcl2 inhibitor navitoclax for 72 h. Comparison of drug response as measured by the area under the curve (AUC) does not show a significant difference between CMS1 and CMS2. Data are shown as mean value ± s.e.m. of 3-4 independent replicates. * p < 0.05, ** p < 0.01, *** p < 0.001, two-sided Student's t-test.
   (D) YM155 induced viability effects are not generally associated with reduced expression of survivin. The CMS1 cells lines RKO and HCT116 were treated with different concentrations of YM155 for 48 h and the expression of survivin was measured by qPCR. Data are shown as mean value ± s.e.m. of 3-4 independent replicates. ** p < 0.01, *** p < 0.001, two-sided Student's t-test.
   (E-F) YM155 induces the expression of genes involved in NFkappaB and ER stress mediated intrinsic apoptosis signalling. (E) HCT116, RKO, HT55 and CaCo2 cells were treated with 10 nM of YM155 for 24 h, after which global expression was determined by microarray analysis. Volcano plot showing increased expression of genes that are associated with NFkappaB (blue) and ER stress mediated intrinsic apoptosis signalling (red). (F) Gene set enrichment analysis of the respective GO terms demonstrate a significant enrichment in YM155 treated cells. (E-F) Expression data from three independent replicates were used.

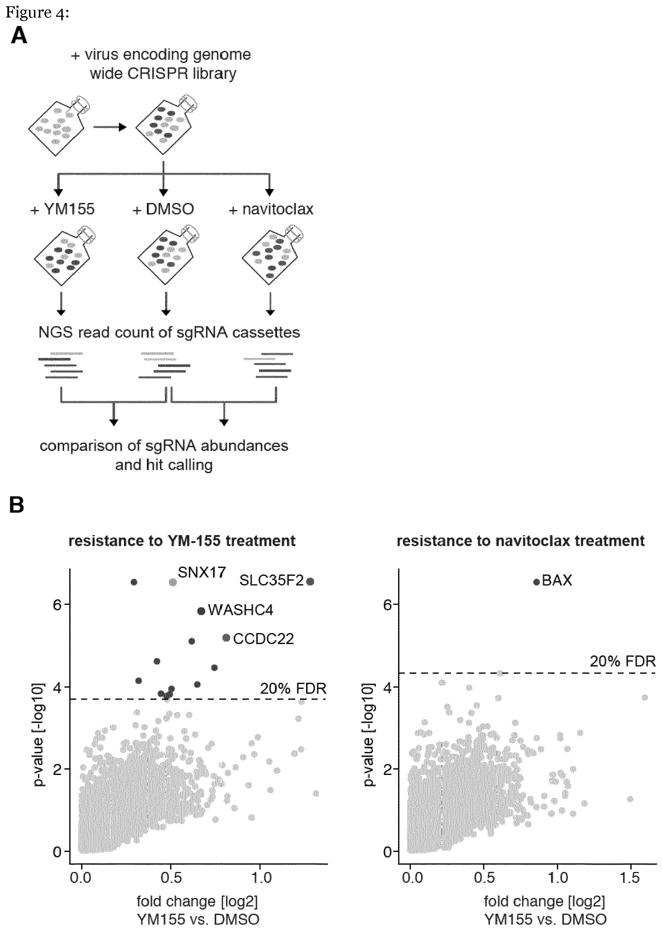

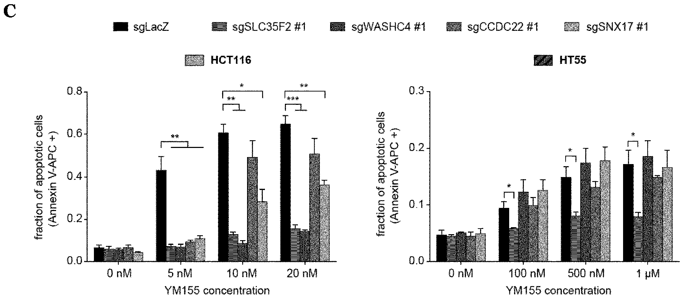

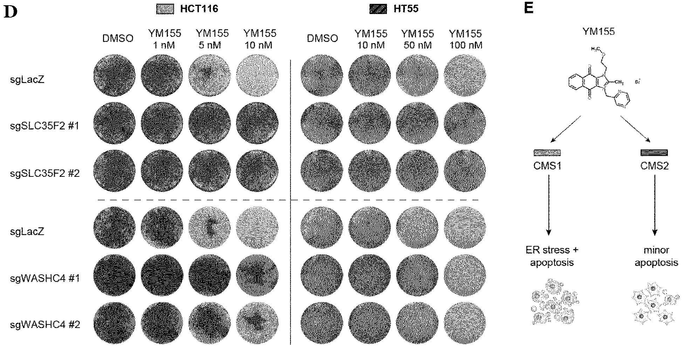

**Figure 4****: A genome-wide CRISPR screen identifies a novel role of LDL receptor endocytosis in YM155 mediated toxicity.**
   (A) Schematic build-up of the genome wide CRISPR screen. In brief, a pooled genome-wide knockout library was generated in HCT116 using the TKO library. After antibiotic selection, the pool was treated with either navitoclax, YM155 or DMSO as control. After 12 d of treatment, cells were harvested and the genomic DNA was isolated. Inserted sgRNA cassettes were amplified by PCR and the relative abundance between conditions was determined by Illumina sequencing.
   (B) Identification of modulators of navitoclax and YM155 response by a pooled CRISPR screen. Volcano plots showing results of the CRISPR screen as analysed by the algorithm MaGECK. The relative fold change of sgRNA abundance of genes is represented by the x-axis while the p-value as calculated by MaGECK is depicted on the y-axis. Only genes with positive fold changes are shown. For YM155, a set of genes involved in LDL-receptor endocytosis (CCDC22, WASHC4 and SNX17) is identified as hits. In comparison, only BAX is identified as a resistance gene in the navitoclax treatment condition.
   (C-D) Validation of candidates by single knockout of candidate genes. The impact of SLC35F2 WASHC4, CCDC22 and SNX17 knockout is validated in the CMS1 cell line HCT116 and the CMS2 cell line HT55 by FACS (C). All candidate genes lead to resistance to YM155 induced apoptosis at different concentrations in HCT116. In contrast, only SLC35F2 knockout mediates resistance in HT55. Data are shown as mean value ± s.e.m of 3 independent replicates. Two independent sgRNAs were selected for validation of each candidate gene. Long-term proliferation assay comparing the effect of YM155 on SLC35F2 and WASHC4 knockout in HCT116 and HT55 (D). Knockout of SLC35F2 induces resistance to YM155 in both cell lines whereas WASHC4 knockout only lead to resistance in HCT116. Representative image of three independent replicates is shown.
   (E) Model of the selective effect of YM155. Treatment with YM155 induces ER stress and strong apoptosis in CMS1 colorectal cancer whereas only minor apoptosis is induced in CMS2 cell lines.

### EXAMPLES

### Example 1: Classification of colorectal cancer cell lines into consensus molecular subtypes

Tumour heterogeneity is a major challenge for the treatment of CRC. Thus, the development of classifiers that predict therapy response is a major line of research. Recently, it was shown that preclinical models of CRC can be classified into CMS, allowing the discovery of subtype specific drug vulnerabilities (Linnekamp et al., 2018; Sveen et al., 2017). Here, the inventors applied a complementary approach by collecting publicly available expression data of 159 CRC cell lines from six independent studies (Barretina et al., 2012; Garnett et al., 2012; Medico et al., 2015; Meyers et al., 2017; Rhodes et al., 2007; Wagner et al., 2007). For each dataset, the CMS of each cell line was separately determined using the R package 'CMSclassifier' (Guinney et al., 2015) (overview in Figure 1A). For many cell lines, transcriptome data was available from multiple sources and the inventors selected the CMS that was supported by the majority of studies. In total, 51% of all cell lines could be assigned to a specific CMS (Figure 1B). The majority was classified as CMS2 (50 cell lines) or CMS1 (22 cell lines), while only few could be assigned to CMS3 or CMS4. In concordance with the distribution of genetic features in primary tumours, the inventors found that CMS1 cell lines have a significantly higher mutation load (Figure 1C) while copy number alterations were more frequent in CMS2 cell lines (Figure 1D). Similarly, the inventors observed a similar distribution of microsatellite instability (Figure 1E) and APC, BRAF, KRAS and TP53 mutations (Figure 1F) between classified cell lines and primary tumours of the same CMS (Guinney et al., 2015). Thus, the analysis underlines that the CMS classification can be applied on a large group of CRC cell lines.

Next, the inventors asked if the CMS can be altered upon engraftment of CRC cell lines into mice. To address this question, the inventors analysed expression data of seven CRC cell lines obtained prior to and at two time-points after subcutaneous engraftment into immune-comprised mice (Hollingshead et al., 2014). For only two cell lines, the inventors observed a shift between CMS1 and CMS2 after prolonged *in vivo* growth, indicating that CMS is a robust cellular feature. Recently, two cell line adapted CMS classifiers were developed (Linnekamp et al., 2018; Sveen et al., 2017) and the inventors compared these algorithms with the original 'CMSclassifier', which the inventors used in the invention. While more CRC cell lines could be assigned to a specific CMS by the adapted classifiers, the inventors observed that especially CMS3 and CMS4 were mostly divergently classified by the three algorithms. Therefore, additional data from functional studies will be needed to support and improve the CMS classification of CRC cell lines.

### Example 2: Identification of subtype specific drug vulnerability to YM155

To discover novel CMS selective compounds, the inventors analysed publicly available data from a large drug screening project (Iorio et al., 2016). Among the CRC cell lines that were included in the screen, 8 could be assigned to CMS1 and 16 to CMS2. For those cell lines, the inventors analysed the viability effect of 256 drugs that cover a broad spectrum of biological targets, using the area under the curve (AUC) as measurement of drug response (see workflow in Figure 2A). Among the identified candidate drugs, YM155 (sepantronium bromide) showed the strongest differential effect between CMS1 and 2 (Figure 2B). YM155 is an antineoplastic drug that was discovered to downregulate the expression of the anti-apoptotic gene survivin (Nakahara et al., 2007). It was tested in phase I and II trials in solid tumours (Giaccone et al., 2009; Satoh et al., 2009), but failed to show clinical benefit despite a good tolerability (Giaccone et al., 2009). For CRC, YM155 has not been tested in clinical studies. To confirm the CMS specific effect of YM155, the inventors determined drug response curves for a panel of CMS1 (HCT116, RKO, LoVo, SNU-C2A, LIM2405, WiDr) and CMS2 (CaCo2, HT55, CL14, SW403, GP2d, HCA24) cell lines, of which most were not included in the previous drug screening project. For each cell line, the inventors confirmed the CMS by an independent expression analysis (not shown). As shown in Figure 2C, CMS1 cell lines were significantly more sensitive towards treatment with YM155 compared to CMS2 counterparts (p = 0.0086, Student's t-test). These results could be confirmed by long-term proliferation assays that show an approximately 100-fold higher sensitivity of CMS1 cell lines (Figure 2D). To validate the specificity of this effect, the inventors treated the same cell lines with 5-fluorouracil and SN-38/irinotecan, which are standard drugs in the treatment of CRC (Figure 2E-F). For those compounds, no difference in response rates could be detected between CMS1 and CMS2. In summary, the inventors identified and independently confirmed that CMS1 CRC is particularly vulnerable to YM155.

### Example 3: YM155 induces apoptosis in CMS1 colorectal cancer by survivin dependent and independent mechanisms

Next, the inventors sought to explore the mechanisms that underlie the vulnerability of CMS1 to YM155. For this purpose, the inventors selected two CMS1 (HCT116, RKO) and CMS2 (HT55, CaCo2) cell lines for subsequent experiments. Since YM155 is a known apoptosis inducing drug, the inventors first measured the percentage of apoptotic cells by flow cytometry after incubation with the compound. In line with the drug response assays, the inventors found that apoptosis was induced in CMS1 cell lines at significantly lower concentrations (Figure 3A). Similarly, Western Blot showed that cleavage of PARP and CASP3 occurred at lower concentrations in RKO compared to CaCo2 (Figure 3B). Since alterations of apoptosis signalling are characteristic for CMS1 CRC (Guinney et al., 2015), the inventors asked if CMS cell lines are also more sensitive to treatment with the BCL-2 inhibitor navitoclax (Tse et al., 2008). However, the inventors found no difference in navitoclax sensitivity between CMS1 and CMS2 cell lines (Figure 3C), indicating that the differential proapoptotic effect is specific to YM155.

Since downregulation of survivin expression is reported to be the main mode of action of YM155 (Nakahara et al., 2007), the inventors asked if differences in survivin levels predict the divergent response to YM155. The inventors compared transcript and protein levels of survivin between CMS1 and CMS2 cell lines using published data (Frejno et al., 2017; Medico et al., 2015), but did not detect any significant differences between subtypes. Next, the inventors sought to investigate if YM155 in fact reduces survivin levels in CRC. The inventors treated four CRC cell lines with YM155 and measured both transcript and protein levels of survivin. The result shows that expression of survivin is only repressed in RKO cells (Figure 3D), while protein levels of survivin are only reduced at high concentrations. Therefore, the findings support the notion that YM155 has a broader range of actions (Rauch et al., 2014). To explore survivin independent mechanisms, the inventors performed global expression analysis of CMS1 and CMS2 cell lines upon YM155 treatment. In contrast to CMS2, global expression was dynamically reshaped upon treatment in CMS1 cell lines (Figure 3E). Amongst the genes that were strongly upregulated, many were associated with ER-stress induced apoptosis (Tabas and Ron, 2011) and NFkappaB signalling (Tabas and Ron, 2011). This observation was confirmed by gene set enrichment analysis which demonstrated significant enrichment of both pathways (Figure 3F). Thus, the inventors show that YM155 preferentially induces apoptosis in CMS1 CRC by mechanisms that are independent of survivin downregulation.

### Example 4: Discovery of novel modifiers of YM155 response by a genome wide CRISPR screen

Next, the inventors aimed to discover novel molecular mechanisms that potentially explain the CMS specific response of YM155. For this purpose, the inventors performed pooled genome-wide CRISPR/Cas9 chemicogenetic screens, which were shown to be a powerful tool for revealing the mode of action of poorly characterized drugs (Gayle et al., 2017; Jost et al., 2017). For the screen, HCT116 was selected since survivin was not downregulated by YM155 in this CMS1 cell line. The inventors generated a genome-wide knockout library using the previously published TKO library (Hart et al., 2015). The pool of knockout cells was then treated with either YM155, the BCL2 inhibitor navitoclax or DMSO (control) for 12 d, after which the abundance of sgRNA cassettes was determined by deep sequencing for each condition (Figure 4A). Quality control of the results demonstrated a high technical quality of the screening experiment. Analysis of the CRISPR screen by the hit calling algorithm MAGeCK (Li et al., 2014) revealed known and novel modifiers of YM155 and navitoclax response (Figure 4B-C). For navitoclax, the screen identified BAX knockout as the main mediator of resistance, which reflects the essential role of BAX as the downstream target of BCL2 (Oltval et al., 1993). In contrast, a larger set of candidates were found to modify the cellular response to YM155, suggesting a more diverse mode of action. Among the top candidates that confer resistance, the inventors identified SLC35F2, which was previously discovered to be the plasma membrane transporter through which YM155 enters the cell (Sutherland et al., 2005). Beyond SLC35F2, the screen uncovered a set of novel resistance genes that are involved in endosomal trafficking of the low-density lipoprotein (LDL) receptor (WASHC4, CCDC22 (Bartuzi et al., 2016), SNX17 (Burden et al., 2004; Stockinger et al., 2002)), indicating a yet unknown connection with YM155 mediated toxicity. To validate the results of the screen, the inventors selected sgRNAs targeting SLC35F2, CCDC22, WASHC4 and SNX17, and performed single knockout experiments in HCT116 and HT55. As control, the inventors selected a previously validated sgRNA against lacZ. Depletion of all the candidate genes resulted in resistance against YM155 induced apoptosis in HCT116, which was similar to the phenotype of SLC35F2 knockout (Figure 4D). This observation could be confirmed by long term proliferation assays, which showed that cells depleted for the candidate genes tolerated higher concentrations of YM155 (Figure 4E). In contrast, knockout of the same genes did not change YM155 sensitivity in HT55. These results indicate that the link between LDL-receptor trafficking and YM155 toxicity could be exclusive for CMS1 CRC and explain the differential vulnerability.

The divergent response to antineoplastic drugs is a major hurdle for the treatment of CRC and demands the discovery of novel markers that reliably predict drug sensitivity. In the present invention, the inventors integrated publicly available expression and drug response data and discovered a novel vulnerability of CMS1 cancers to YM155. Approximately 15% of all CRC can be assigned to CMS1 and a clinical hallmark of this subtype is a very poor prognosis, as recent retrospective analysis of clinical trials demonstrated (Lenz et al., 2017; Stintzing et al., 2017). As most CMS1 cancers show a mismatch-repair deficiency, checkpoint inhibitors have been proposed as a promising treatment option (Le et al., 2015). However, based on the currently limited clinical data, it is yet unknown how many patients will persistently benefit from checkpoint inhibitors. Therefore, the development of alternative or complementary treatment strategies is necessary. YM155 has been tested as a monotherapy and in combination with other drugs for solid, non-GI cancers (Giaccone et al., 2009; Satoh et al., 2009). While it showed a favourable toxicity profile, no clinical benefit was observed. Based on the study, the inventors propose that YM155 is selectively active in CMSi/mismatch deficient cancer and that this association should be further investigated in clinical trials. The increased vulnerability of CMS1 cancer to apoptosis is supported by a recent study which showed that apoptosis signalling is particularly dysregulated in CMS1 and 3 (Lindner et al., 2017). The study also provides novel insights into the mode-of-action of YM155, showing that genes involved in ER-stress mediated apoptosis and NFkappaB signalling are upregulated upon treatment. Since the ER stress response is tightly linked to activation of NFkappaB signalling (Tabas and Ron, 2011), the inventors assume that both mechanisms act together to activate apoptosis. Furthermore, the inventors identify a novel connection between LDL-receptor trafficking and YM155 toxicity using a genome-wide CRISPR screen. A previous study has shown that retention of mutant LDL receptors in the endoplasmic reticulum results in ER stress (Tabas and Ron, 2011). However, the candidate genes that the inventors identified are functionally involved in recycling, but not the synthesis, of the LDL receptor (Bartuzi et al., 2016). Therefore, the data suggest a yet uncharacterized link between ER stress induced apoptosis and endosomal sorting of the LDL receptor, which will need further exploration. Interestingly, both identified mechanisms were exclusively observed in CMS1 cancer, suggesting an Achilles heel that can be exploited by treatment with YM155. In summary, the study shows that combining CMS with drug response profiles in cancer cell lines can uncover novel, subtype-specific vulnerabilities. The results strongly advocate the re-evaluation of YM155 in clinical trials for CMSi/mismatch repair deficient CRC.

### References

Alizadeh, A.A., Aranda, V., Bardelli, A., Blanpain, C., Bock, C., Borowski, C., Caldas, C., Califano, A., Doherty, M., Elsner, M., et al. (2015). Toward understanding and exploiting tumour heterogeneity. Nat. Med. 21, 846-853.
Barretina, J., Caponigro, G., Stransky, N., Venkatesan, K., Margolin, A.A., Kim, S., Wilson, C.J., Lehár, J., Kryukov, G. V., Sonkin, D., et al. (2012). The Cancer Cell Line Encyclopedia enables predictive modelling of anticancer drug sensitivity. Nature 483, 603-607.
Bartuzi, P., Billadeau, D.D., Favier, R., Rong, S., Dekker, D., Fedoseienko, A., Fieten, H., Wijers, M., Levels, J.H., Huijkman, N., et al. (2016). CCC- and WASH-mediated endosomal sorting of LDLR is required for normal clearance of circulating LDL. Nat. Commun. 7, 10961.
Burden, J.J., Sun, X.-M., García, A.B.G., and Soutar, A.K. (2004). Sorting Motifs in the Intracellular Domain of the Low Density Lipoprotein Receptor Interact with a Novel Domain of Sorting Nexin-17. J. Biol. Chem. 279, 16237-16245.
Van Cutsem, E., Cervantes, A., Adam, R., Sobrero, A., Van Krieken, J.H., Aderka, D., Aranda Aguilar, E., Bardelli, A., Benson, A., Bodoky, G., et al. (2016). ESMO consensus guidelines for the management of patients with metastatic colorectal cancer. Ann. Oncol. 27, 1386-1422.
Garnett, M.J., Edelman, E.J., Heidorn, S.J., Greenman, C.D., Dastur, A., Lau, K.W., Greninger, P., Thompson, I.R., Luo, X., Soares, J., et al. (2012). Systematic identification of genomic markers of drug sensitivity in cancer cells. Nature 483, 570-575.
Gayle, S., Landrette, S., Beeharry, N., Conrad, C., Hernandez, M., Beckett, P., Ferguson, S.M., Mandelkern, T., Zheng, M., Xu, T., et al. (2017). Identification of apilimod as a first-in-class PIKfyve kinase inhibitor for treatment of B-cell non-Hodgkin lymphoma. Blood 129, 1768-1778.
Giaccone, G., Zatloukal, P., Roubec, J., Floor, K., Musil, J., Kuta, M., Van Klaveren, R.J., Chaudhary, S., Gunther, A., and Shamsili, S. (2009). Multicenter phase II trial of YM155, a small-molecule suppressor of survivin, in patients with advanced, refractory, non-small-cell lung cancer. J. Clin. Oncol. 27, 4481-4486.
Guinney, J., Dienstmann, R., Wang, X., de Reyniès, A., Schlicker, A., Soneson, C., Marisa, L., Roepman, P., Nyamundanda, G., Angelino, P., et al. (2015). The consensus molecular subtypes of colorectal cancer. Nat. Med. 21, 1350-1356.
Hart, T., Chandrashekhar, M., Aregger, M., Steinhart, Z., Brown, K.R., MacLeod, G., Mis, M., Zimmermann, M., Fradet-Turcotte, A., Sun, S., et al. (2015). High-Resolution CRISPR Screens Reveal Fitness Genes and Genotype-Specific Cancer Liabilities. Cell 163, 1515-1526. Hollingshead, M.G., Stockwin, L.H., Alcoser, S.Y., Newton, D.L., Orsburn, B.C., Bonomi, C.A., Borgel, S.D., Divelbiss, R., Dougherty, K.M., Hager, E.J., et al. (2014). Gene expression profiling of 49 human tumour xenografts from in vitro culture through multiple in vivo passages - strategies for data mining in support of therapeutic studies. BMC Genomics 15, 393. Iorio, F., Knijnenburg, T.A., Vis, D.J., Bignell, G.R., Menden, M.P., Schubert, M., Aben, N., Gonçalves, E., Barthorpe, S., Lightfoot, H., et al. (2016). A Landscape of Pharmacogenomic Interactions in Cancer. Cell 166, 740-754.
Jost, M., Chen, Y., Gilbert, L.A., Horlbeck, M.A., Krenning, L., Menchon, G., Rai, A., Cho, M.Y., Stern, J.J., Prota, A.E., et al. (2017). Combined CRISPRi/a-Based Chemical Genetic Screens Reveal that Rigosertib Is a Microtubule-Destabilizing Agent. Mol. Cell 68, 210-223.e6.
Karapetis, C.S., Khambata-Ford, S., Jonker, D.J., O'Callaghan, C.J., Tu, D., Tebbutt, N.C., Simes, R.J., Chalchal, H., Shapiro, J.D., Robitaille, S., et al. (2008). K-ras Mutations and Benefit from Cetuximab in Advanced Colorectal Cancer. N. Engl. J. Med. 359, 1757-1765.
Le, D.T., Uram, J.N., Wang, H., Bartlett, B.R., Kemberling, H., Eyring, A.D., Skora, A.D., Luber, B.S., Azad, N.S., Laheru, D., et al. (2015). PD-1 Blockade in Tumours with Mismatch-Repair Deficiency. N. Engl. J. Med. 372, 2509-2520.
Lenz, H.-J., Ou, F.-S., Venook, A.P., Hochster, H.S., Niedzwiecki, D., Goldberg, R.M., Mayer, R.J., Bertagnolli, M.M., Blanke, C.D., Zemla, T., et al. (2017). Impact of consensus molecular subtyping (CMS) on overall survival (OS) and progression free survival (PFS) in patients (pts) with metastatic colorectal cancer (mCRC): Analysis of CALGB/SWOG 80405 (Alliance). J. Clin. Oncol. 35, 3511.
Li, W., Xu, H., Xiao, T., Cong, L., Love, M.I., Zhang, F., Irizarry, R. a, Liu, J.S., Brown, M., and Liu, X. (2014). MAGeCK enables robust identification of essential genes from genomescale CRISPR/Cas9 knockout screens. Genome Biol. 15, 554.
Lindner, A.U., Salvucci, M., Morgan, C., Monsefi, N., Resler, A.J., Cremona, M., Curry, S., Toomey, S., O'Byrne, R., Bacon, O., et al. (2017). BCL-2 system analysis identifies high-risk colorectal cancer patients. Gut 66, 2141-2148.
Linnekamp, J.F., Hooff, S.R.V., Prasetyanti, P.R., Kandimalla, R., Buikhuisen, J.Y., Fessler, E., Ramesh, P., Lee, K.A.S.T., Bochove, G.G.W., de Jong, J.H., et al. (2018). Consensus molecular subtypes of colorectal cancer are recapitulated in in vitro and in vivo models. Cell Death Differ. 1-18.
Medico, E., Russo, M., Picco, G., Cancelliere, C., Valtorta, E., Corti, G., Buscarino, M., Isella, C., Lamba, S., Martinoglio, B., et al. (2015). The molecular landscape of colorectal cancer cell lines unveils clinically actionable kinase targets. Nat. Commun. 6, 7002.
Meyers, R.M., Bryan, J.G., McFarland, J.M., Weir, B.A., Sizemore, A.E., Xu, H., Dharia, N. V, Montgomery, P.G., Cowley, G.S., Pantel, S., et al. (2017). Computational correction of copy number effect improves specificity of CRISPR-Cas9 essentiality screens in cancer cells. Nat. Genet. 49, 1779-1784.
Nakahara, T., Takeuchi, M., Kinoyama, I., Minematsu, T., Shirasuna, K., Matsuhisa, A., Kita, A., Tominaga, F., Yamanaka, K., Kudoh, M., et al. (2007). YM155, a novel small-molecule survivin suppressant, induces regression of established human hormone-refractory prostate tumour xenografts. Cancer Res. 67, 8014-8021.
Oltval, Z.N., Milliman, C.L., and Korsmeyer, S.J. (1993). Bcl-2 heterodimerizes in vivo with a conserved homolog, Bax, that accelerates programed cell death. Cell 74, 609-619.
Rauch, A., Hennig, D., Schäfer, C., Wirth, M., Marx, C., Heinzel, T., Schneider, G., and Krämer, O.H. (2014). Survivin and YM155: How faithful is the liaison? Biochim. Biophys. Acta - Rev. Cancer 1845, 202-220.
Rhodes, D.R., Kalyana-Sundaram, S., Mahavisno, V., Varambally, R., Yu, J., Briggs, B.B., Barrette, T.R., Anstet, M.J., Kincead-Beal, C., Kulkarni, P., et al. (2007). Oncomine 3.0: Genes, Pathways, and Networks in a Collection of 18,000 Cancer Gene Expression Profiles. Neoplasia 9, 166-180.
Satoh, T., Okamoto, I., Miyazaki, M., Morinaga, R., Tsuya, A., Hasegawa, Y., Terashima, M., Ueda, S., Fukuoka, M., Ariyoshi, Y., et al. (2009). Phase I study of YM155, a novel survivin suppressant, in patients with advanced solid tumours. Clin. Cancer Res. 15, 3872-3880. Sinicrope, F.A., Okamoto, K., Kasi, P.M., and Kawakami, H. (2016). Molecular Biomarkers in the Personalized Treatment of Colorectal Cancer. Clin. Gastroenterol. Hepatol. 14, 651-658.
Stintzing, S., Wirapati, P., Lenz, H.-J., Neureiter, D., von Weikersthal, L.F., Decker, T., Kiani, A., Vehling-Kaiser, U., Al-Batran, S.-E., Heintges, T., et al. (2017). Consensus molecular subgroups (CMS) of colorectal cancer (CRC) and first-line efficacy of FOLFIRI plus cetuximab or bevacizumab in the FIRE3 (AIO KRK-0306) trial. J. Clin. Oncol. 35, 3510.
Stockinger, W., Sailler, B., Strasser, V., Recheis, B., Fasching, D., Kahr, L., Schneider, W.J., and Nimpf, J. (2002). The PX-domain protein SNX17 interacts with members of the LDL receptor family and modulates endocytosis of the LDL receptor. EMBO J. 21, 4259-4267.
Sutherland, L.C., Rintala-Maki, N.D., White, R.D., and Morin, C.D. (2005). RNA Binding Motif (RBM) proteins: A novel family of apoptosis modulators? J. Cell. Biochem. 94, 5-24.
Sveen, A., Bruun, J., Eide, P.W., Eilertsen, I.A., Ramirez, L., Murumägi, A., Arjama, M.A., Danielsen, S.A., Kryeziu, K., Elez, E., et al. (2017). Colorectal cancer Consensus Molecular Subtypes translated to preclinical models uncover potentially targetable cancer-cell dependencies. Clin. Cancer Res. 24, clincanres.1234.2017.
Tabas, I., and Ron, D. (2011). Integrating the mechanisms of apoptosis induced by endoplasmic reticulum stress. Nat. Cell Biol. 13, 184-190.
Torre, L.A., Bray, F., Siegel, R.L., Ferlay, J., Lortet-Tieulent, J., and Jemal, A. (2015). Global cancer statistics, 2012. CA. Cancer J. Clin. 65, 87-108.
Tse, C., Shoemaker, A.R., Adickes, J., Anderson, M.G., Chen, J., Jin, S., Johnson, E.F., Marsh, K.C., Mitten, M.J., Nimmer, P., et al. (2008). ABT-263: A Potent and Orally Bioavailable Bcl-2 Family Inhibitor. Cancer Res. 68, 3421-3428.
Wagner, K.W., Punnoose, E.A., Januario, T., Lawrence, D.A., Pitti, R.M., Lancaster, K., Lee, D., Von Goetz, M., Yee, S.F., Totpal, K., et al. (2007). Death-receptor O-glycosylation controls tumour-cell sensitivity to the proapoptotic ligand Apo2L/TRAIL. Nat. Med. 13, 1070-1077.

## Claims

1. A compound for use in the treatment of a colorectal cancer (CRC) in a subject, wherein the compound is a fused imidazolium derivative represented by the following general formula (I), and solvates, salts, stereoisomers, complexes, polymorphs, crystalline forms, racemic mixtures, diastereomers, enantiomers, tautomers, isotopically labelled forms, prodrugs, and combinations thereof, 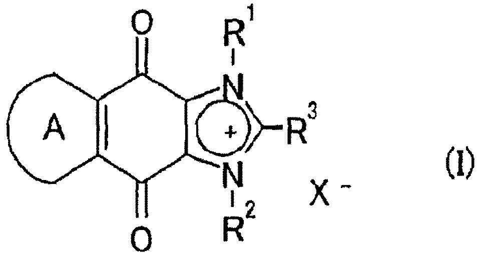 wherein
R¹ and R²: the same or different from each other and each represents -lower alkyl having one or more substituents selected from group B, -lower alkenyl having one or more substituents selected from group B, -lower alkynyl having one or more substituents selected from group B, -RinD, -lower alkyl, -lower alkenyl or -lower alkynyl, with the proviso that at least one of R¹ and R² is -lower alkyl having one or more substituents selected from group B, -lower alkenyl having one or more substituents selected from group B, -lower alkynyl having one or more substituents selected from group B, -cycloalkyl having one or more substituents or -five- to seven-membered saturated heterocyclic ring which may have one or more substituents,
group B: -OR^{a}, -SR^{a}, -prodrug-formed OH, -O-lower alkylene-OR^{a}, -O-lower alkylene-O-lower alkylene-OR^{a}, -O-lower alkylene-NR^{a}R^{b}, -O-lower alkylene-O-lower alkylene-NR^{a}R^{b}, -O-lower alkylene-NR^{c}-lower alkylene-NR^{a}R^{b}, -OCO-NR^{a}R^{b}, - SOR^{a}, -SO₂R^{a}, -SO₂NR^{a}R^{b}, -NR^{a}-SO₂R^{b}, -CO₂H, -NR^{a}R^{b}, -NR^{c}-lower alkylene-NR^{a}R^{b}, -N(-lower alkylene-NR^{a}R^{b})₂, -RinD, -NO₂, -CN, -halogen, -CO₂R^{a}, -COO-, -CONR^{a}R^{b}, -CONR^{a}-O-R^{b}, -NR^{a}-COR^{b}, -NR^{a}-CO-NR^{b}R^{c}, -OCOR^{a} and -CO-R^{a},
R^{a}, R^{b} and R^{c}: the same or different from one another and each represents -H, -lower alkyl, -lower alkylene-RinD or -RinD,
RinD: -five- to seven-membered saturated heterocyclic ring which may have one or more substituents, -cycloalkyl which may have one or more substituents, - cycloalkenyl which may have one or more substituents, -aryl which may have one or more substituents or -heteroaryl which may have one or more substituents,
R³: -H or -lower alkyl which may have one or more substituents, or R² and R³ may together form a lower alkylene having from 2 to 5 carbon atoms which may be interrupted with O, S or NR⁴ (R⁴: -H or -lower alkyl),
ring A: aryl ring which may have one or more substituents or heteroaryl ring which may have one or more substituents, and
X-: counter anion, with the proviso that X- does not exist when the substituent -COO- of the group B forms intramolecular salt with imidazolium cation.

2. The compound for use according to claim 1, wherein A is an unsubstituted aryl.

3. The compound for use according to claim 1 or 2, wherein
(a) R¹ is -CH2(Pyrazin); and/or
(b) R² is -(Ch₂)₂OMe; and/or
(c) R³ is methyl.

4. The compound for use according to any one of claims 1 to 3, wherein X is halogen, preferably Br.

5. The compound for use according to any one of claims 1 to 4, which is 11-(2-methoxyethyl)-2-methyl-4,9-dioxo-3-(pyrazin-2-ylmethyl)-4,9-dihydro-1H-naphtho(2,3-d)imidazolium bromide (YM-155 or SEPANTRONIUM BROMIDE), preferably of the formula (II)

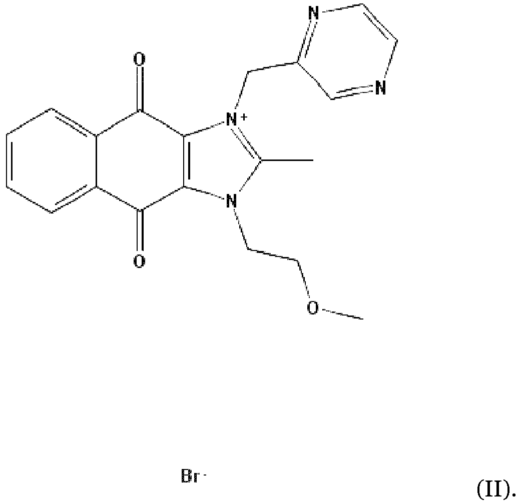

6. The compound for use according to any one of claims 1 to 7, wherein the CRC is a tumour of Consensus Molecular Subtype 1 (CMSi).

7. The compound for use according to claim 6, wherein the tumour is not of CMS2, and most preferably is not any of CMS2, CMS3, and CMS4.

8. The compound for use according to any one of claims 1 to 13, wherein the treatment comprises a prior step of stratifying the subject to be treated by determining the CMS of the CRC tumour in the subject.

9. The compound for use according to claim 8, wherein stratifying involves obtaining a cellular sample of the CRC tumour and determining in said sample an expression level of one or more biomarkers selective and/or specific for a CRC tumour of CMS1.

10. The compound for use according to claim 8 or 9, wherein stratifying involves obtaining a cellular sample of the CRC tumour and determining in said sample the expression, function and/or stability of one or more biomarkers selected from the group consisting of SLC35F2, CCDC22, WASHC4 and SNX17.

11. The compound for use according to any one of claims 1 to 10, wherein the treatment comprises the concomitant or sequential administration of at least one further anti-tumour therapeutic effective for treating CRC to the subject.

12. An *in vitro* method for stratifying a subject suffering from CRC, the method comprising the steps of
(a) Providing a cellular sample of the CRC tumour of the subject,
(b) Determining in said sample any of the following:
(i) the presence or absence of a biomarker in the tumour cells which is selective and/or specific for a CRC tumour of CMS1, and/or
(ii) the expression, function and/or stability of one or more biomarkers selected from the group consisting of SLC35F2, CCDC22, WASHC4 and SNX17 in the tumour cells,
wherein the absence of a biomarker of (i) and/or a reduced expression, function and/or stability of one or more biomarkers of (ii) indicates an increased resistance of the CRC tumour to a treatment with a compound referred to in any one of claims 1 to 5.

13. A method of identifying a whether a patient subgroup suffering from a disorder benefits from a treatment with a candidate compound, comprising
(a) Providing *in vitro* data on the effectiveness of a candidate compound as a therapeutic against a plurality of different cultured cell lines involved with the disorder,
(b) Assigning one or more predetermined consensus molecular subtype(s) of the disorder to each of the different cell lines in (a),
Wherein a significant association of the effectiveness of a candidate compound as a therapeutic with a specific predetermined consensus molecular subtype in the plurality of cultured cell lines indicates that a patient subgroup distinguished by said associated predetermined consensus molecular subtype will benefit from a treatment with said candidate compound.

14. The method according to claim 13, wherein the *in vitro* data is data on the effectiveness of the candidate compound to impair cell viability and/or growth of said *in vitro* cell lines.

15. The method according to claim 13 or 14, wherein the consensus molecular subtype is **characterized by** a differential expression of one or more genes in the cells involved with the disorder compared to at least one other such consensus molecular subtypes of the disorder.
